(19) Europäisches Patentamt
European Patent Office
Office européen des brevets

(11) Veröffentlichungsnummer: **0 120 289**
**B1**

(12) # EUROPÄISCHE PATENTSCHRIFT

(45) Veröffentlichungstag der Patentschrift: **18.05.88**

(21) Anmeldenummer: **84101813.8**

(22) Anmeldetag: **21.02.84**

(51) Int. Cl.⁴: **C 07 D 317/26**, C 07 D 405/14, C 07 D 317/20, C 07 D 317/28, C 07 D 405/04, C 07 D 407/04 // C07D307/32

(54) **Verfahren zur Herstellung von chiralen B-Lactamen.**

(30) Priorität: **25.02.83 CH 1061/83**
**05.01.84 CH 33/84**

(43) Veröffentlichungstag der Anmeldung: **03.10.84 Patentblatt 84/40**

(45) Bekanntmachung des Hinweises auf die Patenterteilung: **18.05.88 Patentblatt 88/20**

(84) Benannte Vertragsstaaten: **AT BE CH DE FR GB IT LI LU NL SE**

(56) Entgegenhaltungen:
**EP-A-0 073 061**
**EP-A-0 096 296**
**EP-A-0 101 598**
**EP-A-0 116 854**
**DE-A-2 541 438**
**US-A-4 111 958**

**CARBOHYDRATE RESEARCH, Band 107, Heft 1, 1982, Elsevier Scientific Publishing Company AMSTERDAM (NL)S. MORGENLIE: "Synthesis of di-0-isopropylidene derivatives of L-fructose", Seiten 137-141.**
**AUSTRALIAN JOURNAL OF CHEMISTRY, Band 31, Heft 6, Juni 1978 MELBOURNE (AU) C. COPELAND et al.: "The Reaction of Isopropenyl Methyl Ether with Aldohexoses and Their Derivatives, Seiten 1371-1374**

(73) Patentinhaber: **F. HOFFMANN- LA ROCHE & CO. Aktiengesellschaft, CH- 4002 Basel (CH)**

(72) Erfinder: **Hubschwerlen, Christian, Dr., Rue de la Gendarmerie, F-68200 Durmenach (FR)**

(74) Vertreter: **Lederer, Franz, Dr., Van der Werth, Lederer & Riederer Patentanwälte Lucile- Grahn-Strasse 22, D-8000 München 80 (DE)**

(56) Entgegenhaltungen: (Fortsetzung)
**CHEMICAL ABSTRACTS, Band 94, Heft 17, 27. April 1981, Seite 736, Zusammenfassung Nr. 139522f Columbus, Ohio (US) S.D. SHARMA et al.: "Some novel monocyclic cis-bêta-lactams from glycine". & Indian J. Chem., Sect. B 1980, 19B(9), 760-4.**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**EP 0 120 289 B1**

LIBER, STOCKHOLM 1988

**Beschreibung**

Die vorliegende Erfindung betrifft ein Verfahren zur Herstellung von chiralen β-Lactamen der allgemeinen Formel

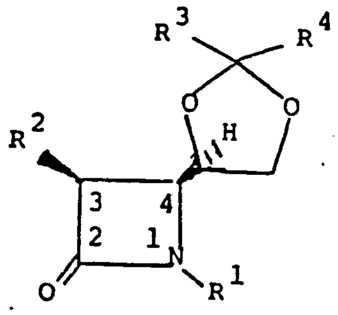

III

in der
$R^1$ eine leicht abspaltbare Schutzgruppe und
$R^2$ Azido, Phthalimido oder die Gruppen ROCO-CH=C($CH_3$)-NH-,
R niederes Alkyl und
$R^3$ und $R^4$ je einzeln Wasserstoff oder niederes Alkyl oder zusammen niederes Alkylen bedeuten,
das dadurch gekennzeichnet ist, dass man ein Lacton der allgemeinen Formel

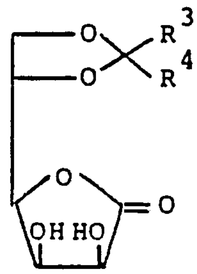

II

worin $R^3$ und $R^4$ obige Bedeutung besitzen,
mit einem Alkalimetallmetaperjodat umsetzt, den erhaltenen Aldehyd der allgemeinen Formel

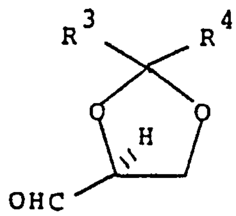

I

worin $R^3$ und $R^4$ obige Bedeutung besitzen,
mit einem Amin der allgemeinen Formel

$H_2N$ - $R^1$ IV

worin $R^1$ obige Bedeutung besitzt, umsetzt, und die so erhaltene Verbindung der allgemeinen Formel

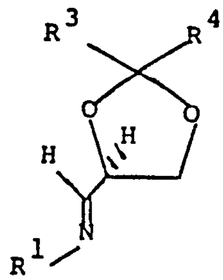

V

worin $R^1$, $R^3$ und $R^4$ obige Bedeutung besitzen,

in Gegenwart einer Base mit einem reaktiven Derivat einer Carbonsäure der allgemeinen Formel

$R^2 - CH_2 - COOH$ VI

worin $R^2$ obige Bedeutung besitzt, umsetzt.

Die im obigen Verfahren eingesetzten Ausgangslactone der allgemeinen Formel II sind neue Verbindungen. Durch die Verwendung dieser neuen Ausgangslactone erhält man in einfacher Weise und mit guter Ausbeute die chiralen Aldehyde der allgemeinen Formel I. Letztere sind für die Synthese von antimikrobiell wirksamen β-Lactamen verwendbar, ferner auch für die Herstellung von chiralem Glycerin bzw. von dessen Acetonid und von Analoga davon. Letztere Produkte sind beispielsweise in der Synthese von Leukotrien (Nachr. Chem. Techn. Lab., 31, 1983, No. 2, S. 117-20), von "Platelet Aggregation Factors" (Helv. Chem. Acta, 1982, S. 1059-84) oder von (R)-γ-Amino-β-hydroxybuttersäure (J. Am. Chem. Soc., 1980, 102, S. 6304-11) verwendbar. In Carbohydrate Research, 107, 137-141 (1982) wird die Herstellung von (S)-Glyceraldehydacetonid durch Oxidation von 5,6-Isopropyliden-L-galactonolacton mit Natriummetaperjodat beschrieben.

Der Ausdruck "niederes Alkyl" bedeutet einen geradkettigen oder verzweigten gesättigten Kohlenwasserstoffrest mit höchstens 4 Kohlenstoffatomen, wie Methyl, Äthyl, Isopropyl und dgl. "Niederes Alkoxy" hat analoge Bedeutung "Niederes Alkylen" betrifft geradkettige oder verzweigte Kohlenwasserstoffreste mit höchstens 6 Kohlenstoffatomen, z. B. Tetramethylen oder, vorzugsweise, Pentamethylen. Der Ausdruck "niederes Alkenyl" bedeutet eine olefinische Kohlenwasserstoffgruppe, die geradkettig oder verzweigt sein kann und bevorzugt bis zu 8, insbesondere bis zu 4 Kohlenstoffatome enthält, wie z. B. Vinyl, 2-Propenyl (Allyl), 1-Propenyl, Isopropenyl, 2-Methallyl, 1- oder 2-Butenyl, 1- oder 2-Hexenyl, 1 oder 2-Heptenyl, 1- oder 2-Octenyl usw.

Im erfindungsgemässen Verfahren wird vorzugsweise das 5,6-Isopropyliden-L-gulonolacton (Verbindung der Formel II, worin $R^3$ und $R^4$ beide Methyl darstellen) eingesetzt. Für die erfindungsgemässe Umsetzung des Ausgangslactons der Formel II mit Alkalimetaperjodat wird vorzugsweise in wässrigen Phase unter Zusatz von Natriummetaperjodat gearbeitet, bevorzugt bei etwa -10° bis +40°C. Vorgängig dieser Umsetzung kann, wahlweise, das Ausgangslacton der Formel II mit Alkalihydroxid, z. B. wässriger Natron- oder Kalilauge zur Spaltung des Lactons (bei etwa 0-10°C) behandelt werden. Die erhaltene, offene Struktur der Formel

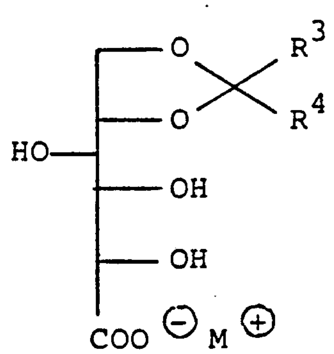

VII

worin $R^3$ und $R^4$ die obige Bedeutung haben und M ein Alkalimetallkation darstellt,

wird neutralisiert, beispielsweise mit wässriger Mineralsäure, z. B. Salzsäure und dann, wie oben, mit dem Alkalimetallperjodat umgesetzt.

Als Amine der Formel IV kommen beispielsweise solche in Betracht, worin $R^1$ eine der folgenden Gruppen darstellt:

- 2,4- oder 3,4-Di-(niederes Alkoxy)benzyl; (a)
- Di-[4-(niederes Alkoxy)-phenyl]-methyl; (b)
- 4-(niederes Alkoxy)-phenyl; (c)
- niederes 2-Alkenyl; (d)
- $CH_2$-$CH(OR^8)_2$; (e)
- 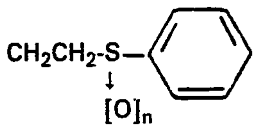 ; (f)

worin $R^8$ niederes Alkyl und n die Zahl 0 oder 1 bedeuten.

Niederes Alkyl ist vorzugsweise Methyl, niederes Alkoxy ist vorzugsweise Methoxy. Niederes 2-Alkenyl ist vorzugsweise 2-Propenyl (Allyl).

Bei der Umsetzung der Verbindungen der Formeln I und IV arbeitet man vorzugsweise bei etwa 0°C bis Raumtemperatur und in einem organisch/wässrigen Zweiphasensystem, z. B. in Methylenchlorid/Wasser, Chloroform/Wasser, Benzol/ Wasser und dgl. Diese Arbeitsweise eignet sich besonders für den Fall, dass die vorangehende Herstellung des Aldehyds der Formel I in wässriger Phase durchgeführt wurde, wobei dieser nicht isoliert zu werden braucht, sondern in der wässrigen Phase direkt weiter umgesetzt werden kann. Isoliert man dagegen den Aldehyd der Formel I, kann dieser mit dem Amin der Formel IV auch in einem organischen Lösungsmittel ohne Wasser umgesetzt werden, beispielsweise in einem halogenierten Kohlenwasserstoff, wie Methylenchlorid, Chloroform, 1,2-Dichloräthan und dergleichen, oder in einem Kohlenwasserstoff, wie Benzol, Toluol und dergleichen. Vorzugsweise entfernt man dabei laufend das während der Reaktion entstehende Reaktionswasser, z. B. durch Arbeiten in Gegenwart eines wasserentziehenden Mittels, beispielsweise in Gegenwart eines geeigneten Molekularsiebes, oder von anderen herkömmlichen Trocknungsmitteln, wie Natriumsulfat, Magnesiumsulfat und dergleichen, vorzugsweise bei Raumtemperatur.

Bei der Reaktion eines reaktiven Derivates einer Carbonsäure der Formel VI mit der erhaltenen Verbindung der Formel V handelt es sich um eine dem Fachmann geläufige Cycloaddition.

Das reaktive Derivat ist vorzugsweise ein entsprechendes Carbonsäurehalogenid, insbesondere ein Carbonsäurechlorid, ein entsprechendes Carbonsäureanhydrid oder ein gemischtes Anhydrid (z. B. mit Trifluoressigsäure, Mesitylensulfonsäure, Chloramaisensäureäthylester, p-Chlorbenzolsulfonsäure und dergleichen), ein entsprechendes Carbonsäureimidazolid und dergleichen. Falls $R^2$ die Gruppe ROCO-CH=$C(CH_3)$-NH- darstellt, kann ein Carbonsäuresalz der Verbindung der Formel VI eingesetzt werden, insbesondere ein Alkalimetallsalz, z. B. das Kaliumsalz, oder ein von tertiären Aminen abgeleitetes Ammoniumsalz, und zwar zusammen mit einem reaktiven organischen Sulfonsäurederivat, insbesondere eines der allgemeinen Formel

$R^9$-$SO_2$-X VIII

worin X Halogen oder die Gruppe -$OSO_2$-$R^9$ und $R^9$ Phenyl, (niederes Alkyl)-phenyl, Halogen-phenyl, niederes Alkyl oder Halogen-niederes-alkyl bedeuten.

Bevorzugte Sulfonsäurederivate sind die Verbindungen der Formel VIII, worin X Halogen, insbesondere Chlor, und $R^9$ Phenyl, (niederes Alkyl)-phenyl oder Halogen-phenyl bedeuten, wie p-Toluolsulfonsäurechlorid, p-Chlorbenzolsulfonsäurechlorid und Benzolsulfonsäurechlorid.

Bei der Umsetzung der Verbindung der Formel V mit einem reaktiven Derivat einer Carbonsäure der Formel VI arbeitet man in Gegenwart einer Base, beispielsweise eines tertiären Amins, wie Triäthylamin oder Diisopropyläthylamin, sowie zweckmässigerweise in einem inerten organischen Lösungsmittel, wobei insbesondere Äther, wie Tetrahydrofuran, Diäthyläther, t-Butylmethyläther, Dioxan, Äthylenglykoldimethyläther oder dergleichen, halogenierte Kohlenwasserstoffe, wie Methylenchlorid, Chloroform, 1,2-Dichloräthan oder dergleichen, Acetonitril, Dimethylformamid oder dergleichen in Frage kommen. Die Umsetzung kann in einem Temperaturbereich von etwa -30°C bis etwa +30°C durchgeführt werden.

Die obige Reaktion eines reaktiven Derivats einer Verbindung der Formel VI mit einer optisch einheitlichen Verbindung der Formel V liefert eine Verbindung der obigen Formel III, wobei die Substituenten in 3- und 4-Stellung des Azetidinonringes zueinander wie erwartet cis-ständig sind. Überraschenderweise hat es sich jedoch gezeigt, dass die Verwendung einer optisch aktiven Verbindung der Formel V in der obigen Cycloaddition in hoher optischer Ausbeute zwei neue optische Zentren induziert, d.h. mit hoher Diastereoselektivität zur Bildung von nur einem von zwei möglichen diastereomeren Produkten führt. Im erhaltenen Produkt konnte das zweite mögliche cis-Cycloadditionsprodukt, das zum tatsächlich erhaltenen Produkt diastereoisomer wäre, nicht nachgewiesen werden.

Die erhaltenen chiralen β-Lactame können, wie erwähnt, für die Herstellung von antimikrobiell wirksamen β-Lactamen verwendet werden, beispielsweise nach den folgenden Reaktionsschemata I und II:

Reaktionsschema I

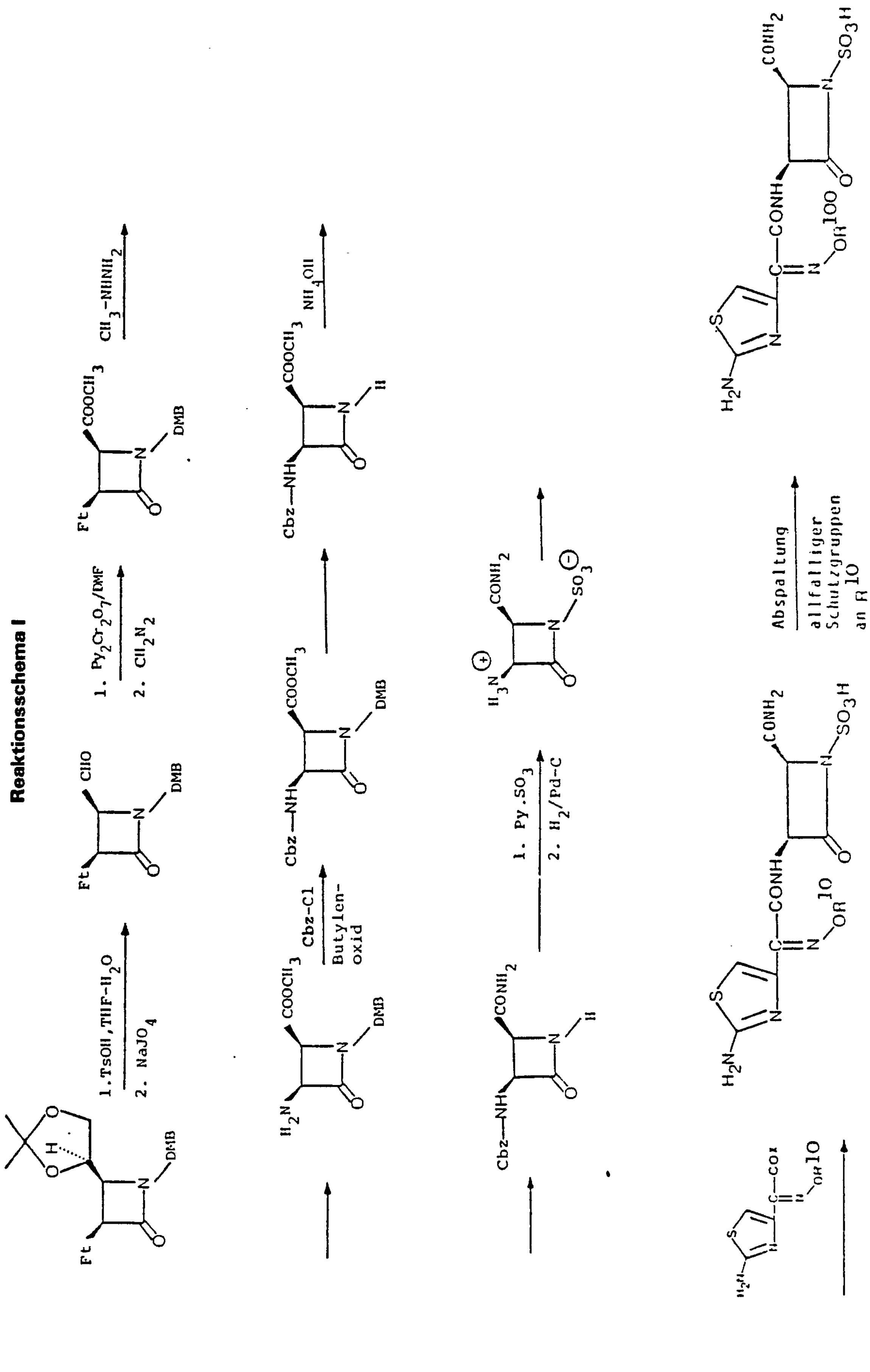

**Reaktionsschema II**

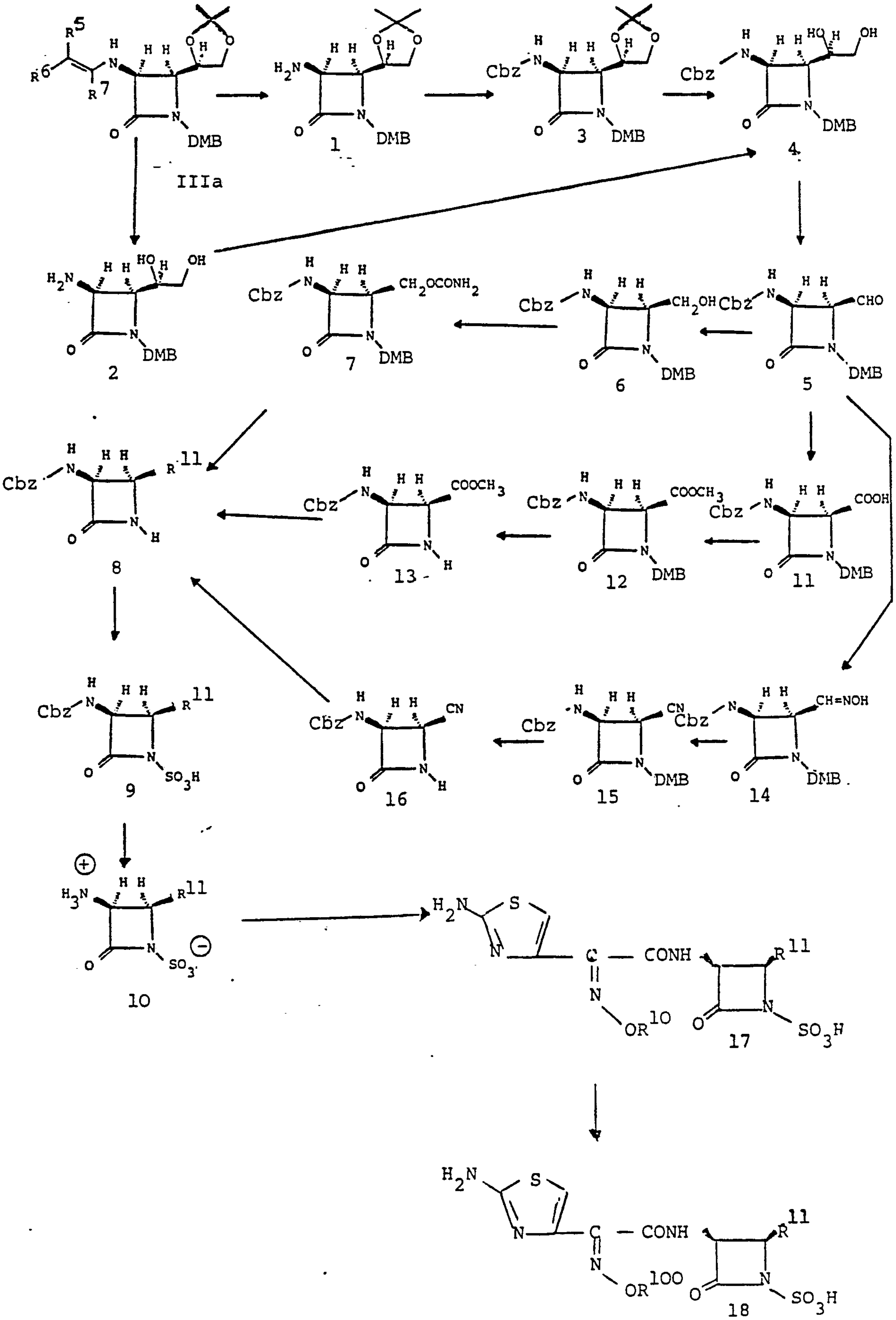

IIIa 1 3 4 2 7 6 5 8 13 12 11 9 16 15 14 10 17 18

Ft = Phthalimido
DMB = 2,4-Dimethoxybenzyl (kann durch andere Schutzgruppen $R^1$ ersetzt werden)
TsOH = p-Toluolsulfonsäure
THF = Tetrahydrofuran
DMF = Dimethylformamid
Py = Pyridin
$Py.SO_3$ = Schwefeltrioxid-pyridiniumkomplex
COX = reaktionsfähiges Derivat einer Carbonsäure, z. B.Säureanhydrid, Säureamid, aktiver Ester, z. B. Benzthiazolylester
$R^{10}$ = Wasserstoff, niederes Alkyl (z. B. Methyl), geschütztes Carboxy-niederes Alkyl, z. B. geschütztes Carboxy-methyl, geschütztes 1-Methyl-1-carboxy-äthyl. Schutzgruppe: z. B. t-Butyl (mit z. B. Trifluoressigsäure abspaltbar), Benzyl und p-Nitrobenzyl (mit z. B. Wasserstoff und Palladiumkohle abspaltbar), 2-(Tri-methylsilyl)-äthyl (mit z. B. Tetrabutylammoniumfluorid abspaltbar)
$R^{100}$ = Wasserstoff, niederes Alkyl (z. B. Methyl), Carboxy-niederes Alkyl, z. B. Carboxymethyl, 1-Methyl-1-carboxy-äthyl
Cbz = Carbobenzoxy (Benzyloxycarbonyl)
$R^{11}$ = Carbamoyl, Carbamoyloxymethyl.

Die Abspaltung der N-Schutzgruppe DMB sowie der obigen Gruppen (a), (b) und (c) der N-Schutzgruppen $R^1$ erfolgt zweckmässigerweise durch milde Oxidation. Ein geeignetes Oxidationsmittel ist Ceriumammoniumnitrat z. B. in wässrigem Acetonitril oder in wässrigem Aceton. 2,4- und 3,4-Di-(nieder-Alkoxy)-benzyl- sowie Di-[4-(nieder-Alkoxy)-phenyl]-methyl-gruppen können ebenfalls mit einem gepufferten Peroxodisulfat, z. B. Ammoniumperoxodisulfat/Ammoniak oder Kaliumperoxodisulfat/Dikaliumhydrogenphosphat, abgespalten werden, wobei man in Wasser und unter annähernd neutralen Bedingungen arbeitet. Di-[4-(nieder-Alkoxy)-phenyl]-methyl-gruppen können auch acidolytisch abgespalten werden, z. B. durch Einwirken von Trifluoressigsäure, Ameisensäure oder Aluminiumchlorid in einem inerten organischen Lösungsmittel, wie Methylenchlorid oder Anisol.

Die Gruppen (d), (e) und (f) der N-Schutzgruppen $R^1$ sind nicht an sich abspaltbar, können aber wie folgt abgespalten werden:

(d): $R^1$ = niederes 2-Alkenyl, kann in niederes 1-Alkenyl übergeführt werden (z. B. $-CH_2-CH=CH_2 \rightarrow -CH=CH-CH_3$).

Diese Isomerisierung erfolgt vorteilhaft mit Hilfe eines Isomerisierungskatalysators, z. B. mit einem Palladiumdihalogenid, wie Palladiumdichlorid oder mit einem Tris(triphenylphosphin)-rhodium(I)-halogenid, z. B. mit dem entsprechenden Chlorid, oder auch mit Palladiumkohle und einer Protonensäure, wie Salzsäure oder Phosphorsäure. Die Isomerisierung wird vorteilhaft in einem inerten organischen Lösungsmittel, wie Äthanol, Methylenchlorid bzw. Mischungen davon mit Wasser, und bei einer Temperatur zwischen etwa 50°C und dem Siedepunkt des Reaktionsgemisches durchgeführt.

Niedere 1-Alkenylgruppen, z. B. 1-Propenyl oder Vinyl, werden oxidativ abgespalten, insbesondere durch Behandeln mit einem Alkalimetallpermanganat, z. B. Kaliumpermanganat, vorzugsweise einer wässrigen Lösung von Kaliumpermanganat. Wahlweise kann man die Oxidation mit Hilfe eines Alkalimetallperjodats, z. B. Kaliumperjodat, in Gegenwart einer katalytischen Menge des erwähnten Alkalimetallpermanganats durchführen. Die Reaktion erfolgt bevorzugt in einem wässrigen gepufferten, isnbesondere auf pH 7-8 gepufferten Medium, kann aber auch in einem mit Wasser mischbaren organischen Lösungsmittel, z. B. in Aceton, Dimethoxyäthan, Dioxan oder Tetrahydrofuran, unter Zusatz einer schwachen organischen Base, wie Pyridin, oder in einer Mischung eines dieser Lösungsmittel mit dem erwähnten wässrigen Puffer, durchgeführt werden. Wahlweise kann die Reaktion ebenfalls unter Phasentransferkatalyse durchgeführt werden, d.h. in Gegenwart einer wässrigen bzw. nicht-wässrigen Phase, z. B. der obige wässrige Puffer sowie ein mit Wasser nicht mischbares, inertes organisches Lösungsmittel, wie z. B. Methylenchlorid oder Benzol. Als Phasentransferkatalysatoren konnen die üblicherweise hierfür verwendeten Agenzien eingesetzt werden, insbesondere organische quaternäre Ammoniumhalogenide, wie Benzyltriäthylammoniumchlorid, Tetra-n-butylammoniumbromid und Cetyltrimethylammoniumbromid. Die oxidative Abspaltung von 1-Alkenylgruppen wird vorzugsweise bei einer Temperatur im Bereiche zwischen etwa 0°C und 25°C durchgeführt.

(e): $R^1 = -CH_2-CH(OR^8)_2 \rightarrow -CH_2-CHO \rightarrow -CO-CH(OH)_2$.

Die erste Umwandlung erfolgt vorzugsweise mit einem Tri-niederalkyljodsilan, insbesondere Trimethyljodsilan, oder mit p-Toluolsulfonsäure. Die Umsetzung erfolgt vorzugsweise in einem inerten organischen Lösungsmittel, wie Acetonitril, und bei einer Temperatur zwischen etwa 0°C und 50°C.

Die zweite Umwandlung (Oxidation) erfolgt vorzugsweise mit Selendioxid unter sauren Bedingungen, z. B. in Gegenwart von Essigsäure. Die Umsetzung erfolgt vorzugsweise in einem inerten organischen Lösungsmittel, wie Dioxan, und bei einer Temperatur zwischen der Raumtemperatur und dem Siedepunkt des Reaktionsgemisches.

Die Dihydroxyacetylgruppe wird bevorzugt mit einer wässrigen starken Base, z. B. mit wässrigem Ammoniak

oder wässriger Alkalilauge, in einem inerten organischen Lösungsmittel, z. B. einem halogenierten Kohlenwasserstoff, wie Chloroform, Kohlenstofftetrachlorid oder Methylenchlorid, bei einer Temperatur zwischen etwa 0° C und +50° C, abgespalten.

(f): $R^1$ =

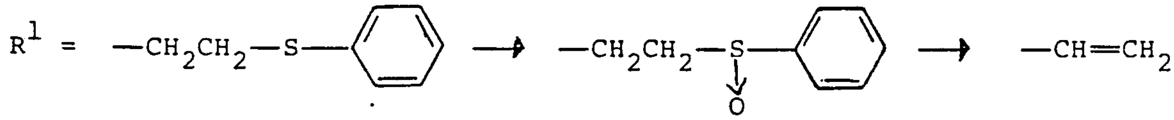

Die erste Stufe, die Oxidation der Phenylthioäthylgruppe zu Phenylsulfinyläthyl, kann durch Behandlung mit einem organischen oder anorganischen Oxidationsmittel erfolgen. Als Oxidationsmittel können verschiedene, Sauerstoff leicht abgebende Verbindungen dienen, wie z. B. organische Peroxide, z. B. monosubstituierte organische Peroxide, wie $C_1$-$C_4$ Alkyl- oder Alkanoylhydroperoxide, wie t-Butylhydroperoxid, Perameisensäure, Peressigsäure; sowie phenylsubstituierte Derivate dieser Hydroperoxide, wie Cumolhydroperoxid, Perbenzoesäure. Der Phenylsubstituent kann gegebenenfalls eine weitere niedere Gruppe, z. B. $C_1$-$C_4$ Alkyl oder Alkoxy, Halogen oder Carboxy, tragen, z. B. 4-Methylperbenzoesäure, 4-Methoxyperbenzoesäure, 3-Chlorperbenzoesäure, Monoperphthalsäure. Als Oxidationsmittel können auch verschiedene anorganische Oxidationsmittel verwendet werden, z. B. Wasserstoffperoxid; Ozon; Permanganate, wie Kalium- oder Natriumpermanganat; Hypochlorite, wie Natrium-, Kalium- oder Ammoniumhypochlorit; Peroxymono- und Peroxydischwefelsäure. Bevorzugt ist die Verwendung von 3-Chlorperbenzoesäure. Die Oxidation erfolgt mit Vorteil in einem inerten Lösungsmittel, z. B. in einem aprotischen inerten Lösungsmittel, wie Tetrahydrofuran, Dioxan, Methylenchlorid, Chloroform, Äthylacetat oder Aceton; oder in einem protischen Lösungsmittel, wie Wasser, einem niederen Alkanol, z. B. Methanol, Äthanol, oder einer niederen ggf. halogenierten Alkancarbonsäure, z. B. Ameisensäure, Essigsäure, Trifluoressigsäure. Die Reaktionstemperatur bewegt sich vornehmlich im Bereiche von etwa -20° C bis +75° C.

Der Abbau der Phenylsulfinyläthylgruppe zur Vinylgruppe erfolgt zweckmässig durch Erhitzen auf etwa 100-200° C, vorzugsweise in einem aprotischen organischen Lösungsmittel, wie Hexamethylenphosphorsäuretriamid, Dimethylsulfoxid oder Dimethylformamid, Benzol oder Toluol. Die Reaktion kann durch Zusatz eines Abfängers für die bei der Reaktion gebildete Phenylsulfonsäure beschleunigt werden, wobei geeignete Agenzien insbesondere Trimethylphosphit oder Propiolsäureester, wie z. B. Propiolsäuremethylester, darstellen.

Die Vinylgruppe wird wie oben unter (d) erläutert abgespalten.

Die in Schema II aufgeführten Reaktionsstufen können wie folgt durchgeführt werden:

Durch milde saure Hydrolyse einer Verbindung der Formel IIIa erhält man eine Verbindung der Formel 1. Die Hydrolyse einer Verbindung der Formel IIIa kann beispielsweise dadurch bewerkstelligt werden, dass man die Verbindung der Formel IIIa in Gegenwart von Wasser und gegebenenfalls einem mit Wasser mischbaren Lösungsmittel, wie Aceton, Tetrahydrofuran, Dioxan, Dimethylsulfoxid, Dimethylformamid oder dergleichen, mit einer Säure behandelt. Als Säuren kommen beispielsweise Mineralsäuren, wie Salzsäure und Schwefelsäure, oder organische Säuren, wie p-Toluolsulfonsäure, Pyridinium-p-toluolsulfonat oder dergleichen, oder schwefelsaure Ionenaustauscher in Frage. Je nach angewendeten Bedingungen wird bei dieser Reaktion auch der Dioxolanring gespalten, wobei man eine Verbindung der Formel 2 erhält.

Durch Behandeln einer Verbindung der Formel 1 oder 2 mit einem die Gruppe Cbz liefernden Mittel erhält man eine Verbindung der Formel 3 bzw. 4. Ein geeigneter, die Gruppe Cbz liefernde Mittel ist beispielsweise der Chlorameisensäurebenzylester. Diese Reaktion erfolgt zweckmässigerweise in einem inerten organischen Lösungsmittel, beispielsweise in einem halogenierten Kohlenwasserstoff, wie Methylenchlorid, Chloroform und dergleichen, und zweckmässigerweise in Gegenwart eines säurebindenden Mittels, wie Butylenoxid, Triäthylamin, Chinuclidin, usw. Zweckmässigerweise arbeitet man bei Raumtemperatur.

Die Hydrolyse einer Verbindung der Formel 3 zu einer Verbindung der Formel 4 erfolgt vorzugsweise unter milden sauren Bedingungen. In einer bevorzugten Ausführungsform bewerkstelligt man die gewünschte Reaktion durch Umacetalisieren in einem niederen Alkohol, wie Methanol oder Äthanol, und in Gegenwart eines geeigneten sauren Katalysators. Geeignete Katalysatoren sind beispielsweise schwefelsaure Ionenaustauscher, Pyridinium-p-toluolsulfonat, p-Toluolsulfonsäure und dergleichen. Man arbeitet dabei vorzugsweise bei Raumtemperatur. Die Hydrolyse kann jedoch ohne weiteres auch in Gegenwart von Wasser und einem mit Wasser mischbaren Lösungsmittel, wie Tetrahydrofuran, Dioxan, Dimethylsulfoxid, Dimethylformamid oder dergleichen, durchgeführt werden.

Die Spaltung der Diolgruppierung in einer Verbindung der Formel 4 erfolgt nach an sich bekannten und jedem Fachmann geläufigen Methoden, und kann beispielsweise mit Natriumperjodat in Wasser bewerkstelligt werden. Gegebenenfalls kann man diese Reaktion in Gegenwart eines Lösungsvermittlers, wie Tetrahydrofuran, Dioxan, Methanol, Äthanol oder dergleichen, durchführen. Man erhält bei dieser Reaktion einen Aldehyd der Formel 5.

Die Reduktion eines Aldehyds der Formel 5 zu einem primären Alkohol der Formel 6 erfolgt ebenfalls nach an sich bekannten und jedem Fachmann geläufigen Methoden, beispielsweise durch Behandeln mit Natriumborhydrid in einem niederen Alkohol, wie Äthanol, Isopropanol oder dergleichen.

Durch Umsetzen einer Verbindung der Formel 6 mit Chlorsulfonylisocyanat in einem inerten organischen Lösungsmittel erhält man eine Verbindung der Formel 7. Geeignete Lösungsmittel sind z. B. Äther, wie

Diäthyläther, t-Butylmethyläther und Äthylenglykoldimethyläther, halogenierte Kohlenwasserstoffe, wie Methylenchlorid und Chloroform, Acetonitril, Dimethylformamid, Dimethylsulfoxid, Aceton und dergleichen. Die Reaktion wird vorzugsweise in einem Temperaturbereich von etwa 0°C bis etwa Raumtemperatur durchgeführt.

Durch Abspalten der mit DMB bezeichneten Schutzgruppe (bzw. anderer Schutzgruppen $R^1$) aus einer Verbindung der Formel 7 erhält man eine entsprechende Verbindung der Formel 8. Die Bedingungen dafür wurden oben beschrieben.

Die Verbindungen der Formel 8, worin $R^{11}$ Carbamoyl bedeutet, können erhalten werden, indem man einen Aldehyd der Formel 5 nach an sich bekannten Methoden zu einer Carbonsäure der Formel 11 oxidiert, die Carbonsäure der Formel 11 beispielsweise mit Methyljodid in Gegenwart von Kaliumcarbonat verestert, aus der erhaltenen Verbindung der Formel 12 die mit DMB bezeichnete Schutzgruppe wie oben beschrieben abspaltet und die erhaltene Verbindung der Formel 13 mit Ammoniak behandelt.

Die Verbindungen der Formel 8, worin $R^{11}$ Carbamoyl bedeutet, können jedoch auch erhalten werden, indem man eine Verbindung der Formel 5 mit Hydroxylamin behandelt, das erhaltene Oxim der Formel 14 in an sich bekannter Weise in das Nitril der Formel 15 überführt, daraus wie oben näher beschrieben die mit DMB bezeichnete Schutzgruppe abspaltet und in der erhaltenen Verbindung der Formel 16 die Nitrilgruppe in an sich bekannter Weise zur Carbamoylgruppe verseift.

Durch Behandeln einer Verbindung der Formel 8 mit Schwefeltrioxid oder einem geeigneten Komplex des Schwefeltrioxids erhält man eine Verbindung der Formel 9. Geeignete Schwefeltrioxidkomplexe sind beispielsweise Komplexe mit Pyridin, Trimethylamin, Picolin, Dimethylformamid und dergleichen. Als Lösungsmittel verwendet man zweckmässigerweise einen Äther, wie Dioxan, Pyridin, Acetonitril, Dimethylformamid oder dergleichen. Acetonitril ist das bevorzugte Lösungsmittel. Die Reaktion wird vorzugsweise zwischen etwa 0°C und 80°C durchgeführt.

Durch Abspaltung der mit Cbz bezeichneten Schutzgruppen aus einer Verbindung der Formel 9 erhält man eine Verbindung der Formel 10. Die Benzyloxycarbonylgruppe Cbz kann beispielsweise hydrogenolytisch abgespalten werden, beispielsweise durch Behandeln mit elementarem Wasserstoff in Gegenwart von Palladium auf Kohle.

Durch Acylierung einer Verbindung der Formel 10 mit einem reaktionsfähigen funktionellen Derivat einer Carbonsäure der allgemeinen Formel

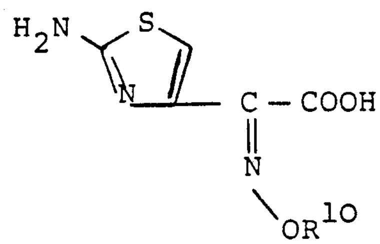

X

worin $R^{10}$ die obige Bedeutung Besitzt,

und Abspaltung allfälliger Schutzgruppen an $R^{10}$ erhält man schliesslich die gewünschte Zielverbindung der Formel 18. Die Verbindung der Formel X muss geeignet geschützt werden, wenn $R^{100}$ in Formel 18 Wasserstoff oder Carboxy-niederes Alkyl bedeuten soll; nach erfolgter Acylierung wird dann die Schutzgruppe entfernt. Als reaktionsfähige funktionelle Derivate von Verbindungen der Formel X kann man beispielsweise entsprechende Säureanhydride, gemischte Säureanhydride, Benzthiazolylthioester und dergleichen verwenden.

Die Zielverbindungen der Formel 18 sind wertvolle Antibiotika mit antibakterieller Wirkung, die zur Bekämpfung und Prophylaxe von Infektionskrankheiten in der Humanmedizin eingesetzt werden können.

Die Überführung der Verbindungen der Formel III, worin $R^2$ Benzyloxycarbonylamino darstellt, in antimikrobiell wertvolle β-Lactam-Antibiotika wird z. B. in der europäischen Patentanmeldung Nr. 82.107 790.6 (Publikation Nr. 0 073 061) und in der europäischen Patentanmeldung Nr. 83.107 910.8 (Publikation Nr. 101 598) beschrieben. Die dabei notwendige Abspaltung von $R^1$ in der Bedeutung "Benzyl" erfolgt in der gleichen Weise wie für $R^1$ als "2,4- bzw. 3,4-Di-(niederes Alkoxy)-benzyl", d.h. oxidativ mit Hilfe eines gepufferten Peroxodisulfats, wie Kaliumperoxodisulfat/Dikaliumhydrogensulfat. Die Benzylgruppe kann aber auch reduktiv durch Einwirken eines Alkalimetalls, z. B. Natrium oder Lithium, in flüssigem Ammoniak abgespalten werden.

Das erfindungsgemäss eingesetzte Lacton der Formel II kann in einfacher und effizienter Weise aus L-Ascorbinsäure hergestellt werden, z. B. indem man diese katalytisch hydriert, z. B. in Gegenwart von Palladiumkohle. Das erhaltene L-Gulonsäure-γ-lacton wird durch Umsetzung mit einem Ketal bzw. Acetal der allgemeinen Formel

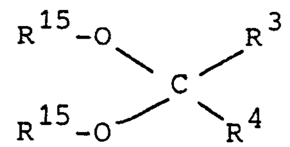

XIII

in der $R^3$ und $R^4$ die oben gegebene Bedeutung haben und $R^{15}$ niederes Alkyl darstellt, in das gewünschte Lacton der Formel II übergeführt. Eine bevorzugte Methode zur Herstellung von 5,6-Isopropyliden-L-gulonolacton (Lacton der Formel II mit $R^3 = R^4$ = Methyl) ist die Umsetzung von L-Gulonsäure-γ-lacton mit Isopropenylmethyläther oder 2,2-(Dimethoxy)-propan. Die Überführung von L-Gulonsäure-γ-lacton in die Ausgangsverbindung der Formel II wird durch Säurezusatz (z. B. durch p-Toluolsulfonsäure) katalysiert. Die Temperatur ist vorzugsweise niedrig, z. B. etwa 0-15° C.

Die nachfolgenden Beispiele erläutern die Erfindung.

**Beispiel 1**

In einen mit Rührer Thermometer Tropftrichter, Glaselektrode und Kühlbad (-15° C, Eis-Methanol) versehenen 10 l Sulfierkolben werden 218 g (1 Mol) 5,6-Isopropyliden-L-gulonolacton in 1 000 ml Wasser suspendiert. Bei +3° C werden innerhalb 30 Minuten 140 ml 50-%-ige wässrige Kalilauge zugetropft. Das Eisbad wird entfernt und das Reaktionsgemisch 15 Minuten bei Raumtemperatur weitergerührt. (Der pH-Wert wird basisch gehalten, und die Vervollständigung der Reaktion wird mittels Dünnschichtchromatogramm kontrolliert). Die braune Lösung wird unter Eiskühlung mit wässriger Salzsäure auf pH 7,0 eingestellt. Die erhaltene gelbe Lösung wird auf -10° C gekühlt und tropfenweise innerhalb einer Stunde mit einer Lösung von 427 g Natriummetaperjodat (2,0 Mol) in 3 000 ml Wasser versetzt. Die Temperatur soll dabei nicht höher als +10° C steigen. Der pH-Wert wird durch Zugabe von gesättigter wässriger Natriumcarbonatlösung auf 5,0 gehalten. Eventuelle Spuren von Jod werden durch Zugabe von Natriumthiosulfat zerstört. Am Ende der Zugabe wird mit gesättigter wässriger Natriumbicarbonatlösung der pH-Wert auf 7,0 eingestellt. Die erhaltene Reaktionslösung enthält rohes (S)-Glyceraldehyd-acetonid; sie wird bei +12° C mit 1 000 ml Methylenchlorid und in einer Argonatmosphäre unter kräftigem Rühren mit einer Lösung von 155 g 2,4-Dimethoxy-benzylamin (0,93 Mol) in 250 ml Methylenchlorid versetzt. Nach 30-minütigem Stehen bei Zimmertemperatur werden die Phasen getrennt, und die Wasserphase wird mit 1 000 ml Methylenchlorid extrahiert. Die vereinigten organischen Phasen werden über 150 g Magnesiumsulfat getrocknet. Die organische Lösung wird filtriert und das Trocknungsmittel mit 300 ml Methylenchlorid nachgewaschen. Die vereinigten organischen Lösungen werden bei 37° C/0,8 mmHg auf etwa 1 l eingedampft. Diese Lösung wird in einen mit Rührer, Thermometer, Chlorcalciumrohr, Tropftrichter und Eis-Methanol-Kühlbad versehenen 2,5 l Sulfierkolben vorgelegt. Dazu werden 174 ml Triäthylamin (124,4 g; 1,23 Mol) gegeben. Nach Kühlung auf 0° C wird innerhalb von 45 Minuten eine Lösung von 207,5 g Phthaloylglycylchlorid (0,93 Mol) in 300 ml Methylenchlorid zugetropft wobei die Temperatur nicht höher als +10° C steigen soll. Das Ganze wird nun bei Zimmertemperatur 2 Stunden gerührt. Nach Überführung in einen Scheidetrichter wird das Ganze nacheinander 3 mal mit 1 000 ml Wasser, 1 mal mit 300 ml wässriger 1N Salzsäure, 2 mal mit 300 ml gesättigter wässriger Natriumbicarbonatlösung, 1 mal mit 500 ml Wasser und schliesslich 1 mal mit 500 ml gesättigter wässriger Natriumchloridlösung gewaschen. Die organische Phase wird über 150 g Natriumsulfat getrocknet, filtriert und zur Trockene eingedampft. Das erhaltene dunkelgelbe, zähe, schaumige Öl wird in 800 ml Äthylacetat gelöst. Die Lösung wird teilweise (um etwa 500 ml) eingeengt und in der Kälte zur Kristallisation gebracht. Man erhält 237 g (53 %) N-[(3S, 4S)-cis-1-(2,4-Dimethoxy-benzyl)-4-[(R)-2,2-dimethyl-1,3-dioxolan-4-yl]-2-oxo-3-azetidinyl]-phthalimid als leicht gelbliche Kristalle vom Schmelzpunkt 155° C.

Das als Ausgangsverbindung verwendete 5,6-Isopropyliden-L-glulonolacton kann wie folgt hergestellt werden:

231 g kristalline L(+)-Ascorbinsäure in 1 700 ml Wasser werden in einem 3 l Kolben katalytisch hydriert (75 g 10 % Pd/C; 65° C; 4 bar $H_2$-Druck; 30 Stunden). Die Reaktionslösung wird filtriert und bei 50° C/12 mmHg zur Trockene eingedampft. Der erhaltene kristalline Rückstand wird in 200 ml siedendes Methanol suspendiert. Die Suspension wird abgekühlt und filtriert. Man erhält 221,6 g (94,8 %) L-Gulonsäure-γ-lacton als farblose Kristalle vom Schmelzpunkt 183-184° C. Die Mutterlauge wird mit 100 ml Äthylacetat verdünnt und 12 Stunden in der Kälte stehen gelassen. Man erhält noch 1 g Produkt, d.h. Totalausbeute 222,6 g (95,3 %).

Eine Lösung von 221,6 g L-Gulonsäure-γ-lacton in 2 000 ml Dimethylformamid wird in einem mit Rührer, Thermometer, Tropftrichter, Chlorcalciumrohr und Eisbad versehenen 3,5 l Sulfierkolben auf 10° C gekühlt und mit 1,8 g p-Toluolsulfonsäure-monohydrat versetzt. Diese Lösung wird tropfenweise bei +10° C mit 116,6 g (154,5 ml; 1,61 Mol) frisch destilliertem Isopropenylmethyläther versetzt. Die erhaltene klare Lösung wird bei 25° C 24 Stunden weiter gerührt. Die so erhaltene farblose Reaktionslösung wird mit 220 g Natriumcarbonat versetzt; die resultierende Suspension wird 2 Stunden weiter gerührt. Die Suspension wird filtriert und die erhaltene Lösung bei 40° C/0,2 mmHg eingeengt. Der erhaltene, leicht gelbliche, kristalline Rückstand wird mit

300 ml Toluol versetzt und unter Eiskühlung mit einem Spatel gerührt. Die gebildeten Kristalle werden abgesaugt, mit wenig Äthanol und viel n-Hexan gewaschen und unter stark vermindertem Druck bei Zimmertemperatur getrocknet. Man erhält 191,6 g (70,9 %) 5,6-Isopropyliden-L-gulonolacton vom Schmelzpunkt 167-168° C.

**Beispiel 2**

43,6 g (0,2 Mol) 5,6-Isopropyliden-L-gulonolacton werden in 200 ml Wasser dispergiert und innerhalb 30 Minuten unter pH Kontrolle (pH 5,5 durch Zugabe von wässriger gesättigter Natriumcarbonatlösung) portionenweise mit 85,5 g (0,4 Mol) Natriummetaperjodat versetzt. Die erhaltene Suspension wird 2 Stunden bei Zimmertemperatur gerührt, mit Natriumchlorid gesättigt und durch Filtration von ausgefällten Natriumjodat befreit. Der pH-Wert des Filtrats wird auf pH 7,0 eingestellt. Die wässrige Lösung von (S)-Glyceraldehyd-acetonid kann wie in Beispiel 1 weiterverarbeitet, oder aber wie folgt isoliert werden:

Die wässrige Lösung wird nacheinander 5 mal mit je 200 ml Methylenchlorid und 4 mal mit je 200 ml Methylacetat extrahiert. Die vereinigten organischen Lösungen werden über Magnesiumsulfat getrocknet und eingedampft. Der Rückstand wird destilliert (Siedepunkt bei 35 mmHg = 64-66°C) wobei man 2,67 g (S)-Glyceraldehyd-acetonid mit 84-%-iger Reinheit und 16,1 g (S)-Glyceraldehyd-acetonid mit 98-%-iger Reinheit erhält. Die totale Ausbeute beträgt 18 g (0,138 Mol 69 %) reines (S)-Glyceraldehyd-acetonid.

**Beispiel 3**

Wird in Beispiel 1 anstelle von 2,4-Dimethoxybenzylamin 3,4-Dimethoxybenzylamin (Veratrylamin) eingesetzt, erhält man unter sonst gleichen Bedingungen N-[(3S,4S)-cis-1-(3,4-Dimethoxybenzyl)-4-[(R)-2,2-dimethyl-1,3-dioxolan-4-yl]-2-oxo-3-azetidinyl]-phthalimid mit IR-Spektrum (KBr): 1 766, 1 720 $cm^{-1}$.

Wird in Beispiel 1 anstelle von 2,4-Dimethoxybenzylamin Benzylamin eingesetzt, erhält man unter sonst gleichen Bedingungen N-[3S,4S)-cis-1-Benzyl-4-[(R)-2,2-dimethyl-1,3-dioxolan-4-yl]-2-oxo-3-azetidinyl]-phthalimid vom Schmelzpunkt 128-129° C.

Wird in Beispiel 1 anstelle von 2,4-Dimethoxybenzylamin 4-Methoxyanilin (p-Anisidin) eingesetzt, erhält man unter sonst gleichen Bedingungen N-[(3S,4S)-cis-1-(4-Methoxyphenyl)-4-[(R)-2,2-dimethyl-1,3-dioxolan-4-yl]-2-oxo-3-azetidinyl]-phthalimid vom Schmelzpunkt 64,7° C; $\alpha^{20}_D = +20{,}3°$ (c = 1 in Methanol).

Wird in Beispiel 1 anstelle von 2,4-Dimethoxybenzylamin 4-Methoxybenzylamin eingesetzt, erhält man unter sonst gleichen Bedingungen N-[(3S,4S)-cis-1-(4-Methoxybenzyl)-4-[(R)-2,2-dimethyl-1,3-dioxolan-4-yl]-2-oxo-3-azetidinyl]phthalimid mit IR-Spektrum (KBr): 1766, 1721 $cm^{-1}$; $[\alpha]^{20}_D = + 63{,}8°$ (c = 1 in Chloroform).

Elementaranalyse:

| | | | |
|---|---|---|---|
| Berechnet: | C 66,05 | H 5,54 | N 6,42 % |
| Gefunden: | C 65,82 | H 5,61 | N 6,29 % |

**Beispiel 4**

a) Man löst 12 g (92,16 mMol) (S)-Glyceraldehyd-acetonid in 200 ml Methylenchlorid, versetzt zuerst mit 60 g Magnesiumsulfat und dann tropfenweise über einen Zeitraum von 10 Minuten mit 5,26 g (92,16 mMol) 3-Amino-1-propen in 50 ml Methylenchlorid und rührt die Suspension während 5 Stunden bei Raumtemperatur. Man filtriert das Magnesiumsulfat ab und dampft die klare farblose Lösung am Rotationsverdampfer ein. Man erhält 14,6 g (86,3 mMol; 93,6 %) reines (S)-Glyceraldehyd-acetonid-allyl-imin.

b) Man löst 8,64 g (51,1 mMol) (S)-Glyceraldehyd-acetonid-allyl-imin in 400 ml Methylenchlorid, versetzt zuerst mit 10,32 g (102,2 mMol) Triäthylamin und dann mit 10,79 g (51,1 mMol) N-(1-Methyl-2-methoxycarbonylvinyl)aminoessigsäure-Kaliumsalz, kühlt die Suspension auf 0° C ab und versetzt tropfenweise innerhalb von 5 Minuten mit 9,74 g (51,1 mMol) p-Toluolsulfonsäurechlorid in 50 ml Methylenchlorid. Man entfernt das Kühlbad, rührt das Reaktionsgemisch während 8 Stunden bei Raumtemperatur, versetzt mit 200 ml Wasser, trennt die organische Phase ab und dampft sie ein. Das Rohprodukt wird an Kieselgel unter Eluieren mit Hexan/Essigester (8 : 2) chromatographiert. Man erhält 13,0 g (40,0 mMol; 78 %) Methyl (Z)-3-[[(3S,4S)-1-allyl-4-[(R)-2,2-dimethyl-1,3-dioxolan-4-yl]-2-oxo-3-azetidinyl]amino]-2-butenoat als Öl, das beim Stehenlassen langsam erstarrt. Durch Kristallisieren aus Äther/Hexan erhält man ein Produkt vom Schmelzpunkt 98° C.

**Beispiel 5**

Man löst 3,75 g (13,44 mMol) (S)-Glyceraldehyd-acetonid-(2,4-dimethoxybenzyl)imin (erhalten gemäss Angaben von Beispiel 1a) aus (S)-Glyceraldehyd-acetonid und 2,4-Dimethoxybenzylamin) in 150 ml Methylenchlorid und versetzt mit 3,25 g (32,25 mMol) Triäthylamin. 3,40 g (16,12 mMol) N-(1-Methyl-2-methoxycarbonylvinyl) aminoessigsäure-Kaliumsalz werden zugegeben und die Suspension wird auf 0°C abgekühlt. Man tropft innerhalb von 5 Minuten 3,40 g (16,12 mMol) p-Toluolsulfonsäurechlorid in 50 ml Methylenchlorid dazu und rührt das Gemisch während 15 Stunden bei Raumtemperatur. Nach Waschen mit 100 ml Wasser und Eindampfen der organischen Phase wird der Rückstand durch Chromatographieren an Kieselgel mit Hexan/Essigester (7:3) als Elutionsmittel gereinigt, und man erhält 3,45 g (7,94 mMol; 59 %) reines Methyl (Z)-3-[[(3S,4S)-1-(2,4-dimethoxybenzyl)-4-[(R)-2,2-dimethyl-1,3-dioxolan-4-yl]-2-oxo-3-azetidinyl]amino]-2-butenoat als Öl, das langsam erstarrt. Nach Kristallisieren aus Äther/Hexan hat das Produkt einen Schmelzpunkt von 121°C.

**Beispiel 6**

Man löst 6,48 g (20 mMol) Methyl (Z)-3-[[(3S,4S)-1-allyl-4-[(R)-2,2-dimethyl-1,3-dioxolan-4-yl]-2-oxo-3-azetidinyl ]amino]-2-butenoat in 40 ml Aceton und versetzt mit 3,80 g (20 mMol) p-Toluolsulfonsäure-monohydrat in 20 ml Aceton. Die klare Lösung wird während 15 Minuten bei Raumtemperatur gerührt und dann langsam mit 120 ml Äther versetzt. Das ausgefallene Produkt wird abfiltriert und getrocknet. Man erhält 6,4 g (15,3 mMol; 77 %) reines (3S,4S)-cis-3-Amino-1-allyl-4-[(R)-2,2-dimethyl-1,3-dioxolan-4-yl]-2-azetidinon-p-Toluolsulfonsäuresalz vom Schmelzpunkt 165°C.

**Patentansprüche**

1. Verfahren zur Herstellung von chiralen β-Lactamen der allgemeinen Formel

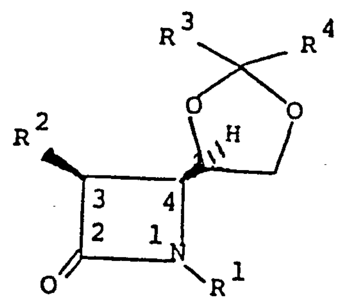

III

worin $R^1$ eine leicht abspaltbare Schutzgruppe, $R^2$ Azido, Phthalimido oder die gruppe ROCO-CH=C($CH_3$)-NH-, R niederes Alkyl und $R^3$ und $R^4$ je einzeln Wasserstoff oder niederes Alkyl oder zusammen Alkylen mit höchstens 6 Kohlenstoffatomen bedeuten, und die mit nieder bezeichneten Gruppen höchstens 4 Kohlenstoffatome besitzen, dadurch gekennzeichnet, dass man ein Lacton der allgemeinen Formel

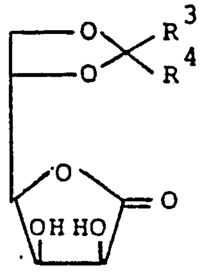

II

worin $R^3$ und $R^4$ obige Bedeutung besitzen, mit einem Alkalimetallmetaperjodat umsetzt, den erhaltenen Aldehyd der allgemeinen Formel

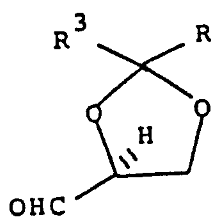

I

worin $R^3$ und $R^4$ obige Bedeutung besitzen, mit einem Amin der allgemeinen Formel

$H_2N\text{-}R^1$ IV

worin $R^1$ obige Bedeutung besitzt, umsetzt, und die so erhaltene Verbindung der allgemeinen Formel

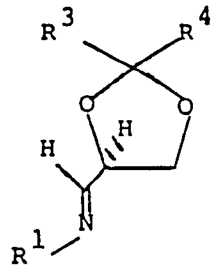

V

worin $R^1$, $R^3$ und $R^4$ obige Bedeutung besitzen, in Gegenwart einer Base mit einem reaktiven Derivat einer Carbonsäure der allgemeinen Formel

$R^2\text{-}CH_2\text{-}COOH$ VI

worin $R^2$ obige Bedeutung besitzt, umsetzt.

2. Verfahren nach Anspruch 1, dadurch gekennzeichnet, dass man als Ausgangsverbindung der allgemeinen Formel II 5,6-Isopropyliden-L-gulonolacton verwendet.

3. Verfahren nach Anspruch 1 oder 2, dadurch gekennzeichnet, dass man die Umsetzung der Verbindungen der allgemeinen Formeln I und IV in einem organisch/wässrigen Zweiphasensystem durchführt.

4. Verfahren nach Anspruch 3, dadurch gekennzeichnet, dass man als organische Komponente für das organisch/wässrige Zweiphasensystem Methylenchlorid verwendet.

5. Verfahren nach einem der Ansprüche 1-4, dadurch gekennzeichnet, dass man ein Ausgangsamin der Formel IV verwendet, worin $R^1$ eine der folgenden Gruppen bedeutet:

- 2,4- oder 3,4-Di (niederes Alkoxy)benzyl (a);
- Di[4-(niederes Alkoxy)phenyl]methyl (b);
- 4-(niederes Alkoxy)phenyl (c);
- 2-Alkenyl mit bis zu 8 Kohlenstoffatomen (d);
- $-CH_2\text{-}CH(OR^8)2$ (e) oder
- 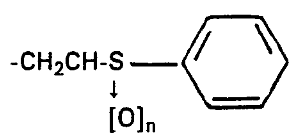 (f) ; (f);

worin $R^8$ niederes Alkyl und n die Zahl 0 oder 1 bedeuten, und die mit nieder bezeichneten Gruppen höchetens 4 Kohlenstoffatome besitzen.

6. Verfahren nach einem der Ansprüche 2-5, dadurch gekennzeichnet, dass man das Säurechlorid der Carbonsäure der Formel VI einsetzt.

7. Verfahren nach einem der Ansprüche 2-6, dadurch gekennzeichnet, dass man ein reaktives Derivat von Phthaloylglycin verwendet.

**Claims**

1. A process for the manufacture of chiral β-lactams of the general formula

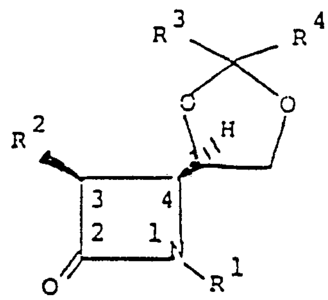

III

wherein $R^1$ signifies a readily cleavable protecting group, $R^2$ signifies azido, phthalimido or the group ROCO-CH=C($CH_3$)-NH-, R signifies lower alkyl and $R^3$ and $R^4$ each individually signify hydrogen or lower alkyl or together signify alkylene with a maximum of 6 carbon atoms, and the groups denoted by lower have a maximum of 4 carbon atoms, characterized by reacting a lactone of the general formula

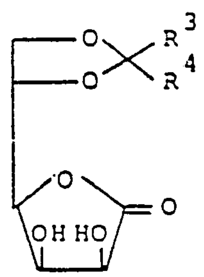

II

wherein $R^3$ and $R^4$ have the above significance, with an alkali metal periodate, reacting the resulting aldehyde of the general formula

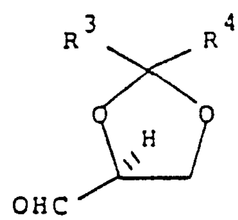

I

wherein $R^3$ and $R^4$ have the above significance, with an amine of the general formula

$H_2N\text{-}R^1$ IV

wherein $R^1$ has the above significance, and reacting the thus-obtained compound of the general formula

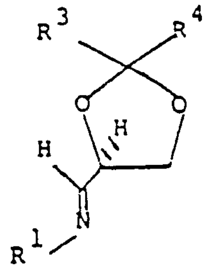

V

wherein $R^1$, $R^3$ and $R^4$ have the above significance, in the presence of a base with a reactive derivative of a carboxylic acid of the general formula

$R^2$-$CH_2$-COOH H VI

wherein $R^2$ has the above significance.

2. A process according to claim 1, characterized in that 5,6-isopropylidene-L-gulonolactone is used as the starting compound of general formula II.

3. A process according to claim 1 or 2, characterized in that the reaction of the compounds of general formulae I and IV is carried out in an organic/aqueous two-phase system.

4. A process according to claim 3, characterized in that methylene chloride is used as the organic component for the organic/aqueous two-phase system.

5. A process according to any one of claims 1-4, characterized in that there is used a starting amine of formula IV in which $R^1$ signifies one of the following groups:

- 2,4- or 3,4-di(lower alkoxy)benzyl (a);
- di[4-(lower alkoxy)phenyl]methyl (b);
- 4-(lower alkoxy)phenyl (c);
- 2-alkenyl with up to 8 carbon atoms (d);
- $-CH_2-CH(OR^8)_2$ (e) or
- 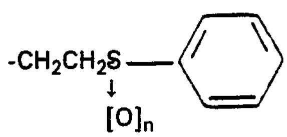 (f); (f);

wherein $R^8$ signifies lower alkyl and n signifies the number 0 or 1, and the groups denoted by lower have a maximum of 4 carbon atoms.

6. A process according to any one of claims 2-5, characterized in that the acid chloride of the carboxylic acid of formula VI is used.

7. A process according to any one of claims 2-6, characterized in that a reactive derivative of phthaloylglycine is used.

**Revendications**

1. Procédé de préparation de β-lactames chiraux de formule générale:

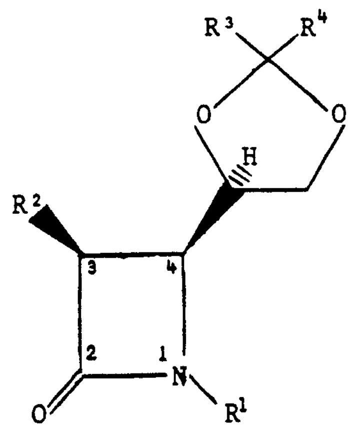

III

dans laquelle $R^1$ représente un groupe protecteur facile à éliminer, $R^2$ représente un groupe azido, phtalimido ou le groupe ROCO-CH=C($CH_3$)-NH-, R représente un groupe alkyle inférieur, et $R^3$ et $R^4$ représentent chacun, indépendamment l'un de l'autre, l'hydrogène ou un groupe alkyle inférieur ou bien forment ensemble un groupe alkylène contenant au maximum 6 atomes de carbone, les groupes qualifiés d'"inférieurs" contenant au maximum 4 atomes de carbone, caractérisé en ce que l'on fait réagir une lactone de formule générale:

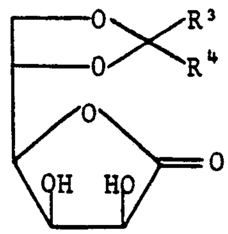

II

dans laquelle $R^3$ et $R^4$ ont les significations indiquées ci-dessus, avec un métaperiodate de métal alcalin, ce qui donne un aldéhyde de formule générale:

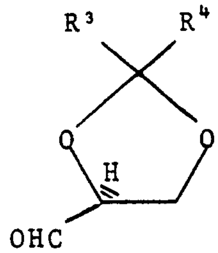

I

dans laquelle $R^3$ et $R^4$ ont les significations indiquées ci-dessus, qu'on fait réagir avec une amine de formule générale:

$H_2N-R^1$ IV

dans laquelle $R^1$ a les significations indiquées ci-dessus, ce qui donne un composé de formule générale:

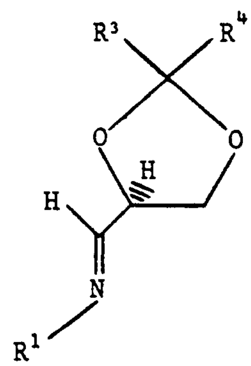

V

dans laquelle $R^1$ $R^3$ et $R^4$ ont les significations indiquées ci-dessus, qu'on fait réagir en présence d'une base avec un dérivé réactif d'un acide carboxylique de formule générale:

$R^2-CH_2-COOH$ VI

dans laquelle $R^2$ a les significations indiquées ci-dessus.

2. Procédé selon la revendication 1, caractérisé en ce que l'on utilise en tant que composé de départ de formule générale II la 5,6-isopropylidène-L-gulonolactone.

3. Procédé selon la revendication 1 ou 2, caractérisé en ce que la réaction entre les composés de formules générales I et IV est effectuée dans un système organique/ aqueux à deux phases.

4. Procédé selon la revendication 3, caractérisé en ce que l'on utilise en tant que composant organique du système organique/aqueux à deux phases le chlorure de méthylène.

5. Procédé selon l'une des revendications 1 à 4, caractérisé en ce que l'on utilise une amine de départ de formule IV dans laquelle $R^1$ représente l'un des groupes suivants:

- 2,4- ou 3,4-di-(alcoxy inferieur)-benzyle (a)
- di-[4-(alcoxy inférieur)-phényl]-méthyle (b)
- 4-(alcoxy inférieur)-phényle (c)
- 2-alcényle contenant jusqu'à 8 atomes de carbone (d)
- $-CH_2-CH(OR_8)_2$ (e)

ou

- 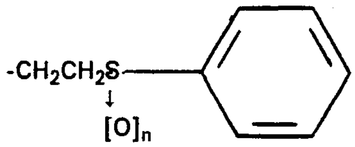 (f)

dans laquelle $R^8$ représente un groupe alkyle inférieur et n est égal à 0 ou 1, et les groupes qualifiés d'"inférieurs" contiennent au maximum 4 atomes de carbone.

6. Procédé selon l'une des revendications 2 à 5 caractérisé en ce que l'on utilise le chlorure d'acide de l'acide carboxylique de formule VI.

7. Procédé selon l'une des revendications 2 à 6, caractérisé en ce que l'on utilise un dérivé réactif de la phtaloylglycine.